# EUROPEAN PATENT SPECIFICATION

(11) **EP 4 398 748 B1**
(45) Date of publication and mention of the grant of the patent: **11.06.2025**
(21) Application number: 22773308.6
(22) Date of filing: 02.09.2022
(51) Int. Cl.: A24F 40/20, A24F 40/42, A24F 40/85, H05B 3/00, H05B 3/04, H05B 3/06, A61M 11/04, A61M 15/00, A61M 15/06

(54) **RESISTIVE HEATING AEROSOL-GENERATING SYSTEM WITH NICOTINE TAPE**
AEROSOLERZEUGENDES SYSTEM MIT WIDERSTANDSHEIZUNG UND NIKOTINBAND
SYSTÈME DE GÉNÉRATION D'AÉROSOL À CHAUFFAGE RÉSISTIF COMPORTANT UNE BANDE DE NICOTINE

(30) Priority: 09.09.2021 EP 21195768
(43) Date of publication of application: 17.07.2024
(73) Proprietor: Philip Morris Products S.A., 2000 Neuchâtel (CH)
(72) Inventor: FREDERICK, Guillaume, 2000 Neuchâtel (CH); MIRONOV, Oleg, 2000 Neuchâtel (CH); TAURINO, Irene, 2000 Neuchâtel (CH)
(74) Representative: Abitz & Partner
(86) International application number: PCT/IB2022/058267
(87) International publication number: WO 2023/037213

(56) References cited:
- EP-A1- 0 640 297
- EP-A1- 3 068 246
- WO-A1-2020/207734
- WO-A1-2021/042359
- WO-A1-2021/099288
- US-A- 2 624 925
- DATABASE WPI Week 202104, Derwent World Patents Index; AN 2021-58514F, XP002805792
- DATABASE WPI Week 2021, Derwent World Patents Index; AN 2021-01394P, XP002805793

## Description

The present disclosure relates to an aerosol-generating system with nicotine tape, the aerosol-generating system is configured to generate aerosol via resistive heating of the nicotine tape.

Sensorial media of commercial electronic cigarettes are generally liquid (so called "e-liquids"). These e-liquids are typically contained in aerosol-generating articles or "pods" that are replaceable into the electronic cigarette. These pods include a heater in contact with a porous material or wick. This porous material or wick transports e-liquid by capillarity action to the heater for vaporization into inhalation airflow of the electronic cigarettes.

A well-known problem of commercial electronic cigarettes is e-liquid leakage which typically occurs when: there is too much e-liquid; the retention capability of the porous material does not prevent the e-liquid from flooding especially in the case of full tank commercial electronic cigarettes; changes occur in environmental pressure during storage or transport; or temperature changes which affects the viscosity of the e-liquid or the retention capacity of the porous materials.

In addition, the heater of current commercial electronic cigarette systems is typically integrated in the replaceable cartridge or "pod" containing the e-liquid. Including the heater in the replaceable aerosol-generating article or "pod" increases the cost, complexity, and disposal costs of these replaceable aerosol-generating articles or "pods".

Document CN 112 806 617 A relates to a gel-state tobacco tar electronic cigarette. The gel-state tobacco tar electronic cigarette comprises: a smoking set shell, wherein a control circuit board, a battery and a smog channel are arranged in the box body; and a placing reel, wherein a gel state tobacco tar strip is wound on the placing reel, the gel state tobacco tar strip is unwound from the placing reel and wound by a driving reel after being turned by a driven reel, a fan-shaped resistance heating layer is arranged on the circumferential outer surface of the driven reel, the gel-state tobacco tar strip comprises a strip fuming section and a strip non-fuming section which are arranged at an interval, and the length of each strip fuming section is matched with the arc length of the fan-shaped subarea of the resistance heating layer on the circumferential outer surface. According to the electronic cigarette, smoking and smoking intervals can be simulated under continuous working of the micromotor, smoking habits of smokers are met, and the service life of a micromotor is prolonged.

Document CN 112 120 290 A relates to an atomization device, which comprises an oil bin, an atomizer and a circulating moving liquid suction mechanism, cigarette liquid is stored in the oil bin, a heating device is arranged in the atomizer, the circulating moving liquid suction mechanism comprises a driving structure and a liquid suction piece coupled with the driving structure, the driving structure is used for driving the liquid suction piece to move, wherein a moving path of the liquid absorbing piece circularly passes through the heating ranges of the oil bin and the heating device, the liquid absorbing piece absorbs tobacco liquid at the oil bin, and the absorbed tobacco liquid is brought to the heating device by virtue of the movement of the liquid absorbing piece to be heated and atomized; wherein the moving path of the liquid absorbing part is arranged in a two-dimensional plane or a three-dimensional moving path. According to the atomization device, the amount of tobacco juice conveyed to the heating device can be adjusted and controlled more easily, and therefore adverse effects caused by excessive or insufficient conveying of the tobacco juice are avoided.

Document WO 2021/099288 A1 relates to a vaporizer comprising a mouth piece the mouth piece being in fluid connection with a vaporizing section, wherein the vaporizing section comprises at least a first vaporizable substance and a heater to heat the vaporizable substance. The vaporizer comprises a substance cartridge comprising the at least one vaporizable substance, wherein the vaporizable substance is provided as a substance section on or as a tape, and the substance section is arranged movable in a predefined transport direction in the substance cartridge.

Document EP 3 068 246 A1 relates to a device for generating an inhalable aerosol and/or gas, the device comprising an aerosol generating material having an integrated electrical resistance heating element, so that the aerosol generating material may be heated in direct contact with the electrical resistance heating element, wherein the aerosol generating material is provided as a unitary structure and/or coating which may be heated to generate multiple deliveries of an inhalable aerosol and/ or gas. There is also provided a method for fabricating the device. There is also provided the use of the device and the use of the aerosol generating material, to generate an inhalable aerosol and/or gas.

Document US 2 624 925 A relates to vaporizers, more particularly to a device for vaporizing air sterilizing materials, such as glycols. In another aspect, the document relates to a charge for such vaporizers including an absorbent carrier impregnated with a material to be vaporized and free from unabsorbed vaporizable, material.

Document WO 2020/207734 A1 relates to an aerosol-generating system comprising: an aerosol-generating film comprising a cellulose based film-forming agent, a non-cellulose based thickening agent and a polyhydric alcohol; a heater assembly for heating the aerosol-generating film to generate an inhalable aerosol; a power supply; and control circuitry configured to control a supply of power from the power supply to the heating assembly in response to a user inhalation, wherein the control circuitry is configured to activate the heater assembly to sequentially heat different portions of the aerosol-generating film in response to successive user inhalations. An aerosol-generating article comprising: a support; and a plurality of spaced-apart discrete portions of aerosol- generating film disposed on the support, wherein each spaced-apart discrete portion of aerosol-generating film comprises a cellulose based film-forming agent, a non-cellulose based thickening agent and a polyhydric alcohol.

Document WO 2021/042359 A1 relates to a heat-not-burn tobacco paper cartridge including a first rotatable support and a second rotatable support. The first and second rotatable supports are configured to transfer a strip of tobacco paper from the first rotatable support to the second rotatable support. The cartridge also includes a heater positioned between the first and second rotatable supports. The heater is configured to heat a portion of the strip of tobacco paper extending between the first and second rotatable supports.

Document EP 0 640 297 A1 relates to an electrical smoking article in which tobacco flavor medium is carried past a heater by a continuous web. The web, which preferably is substantially non-combustible, may bear continuous tobacco flavor medium or spaced-apart portions of tobacco flavor medium. A flavor cassette for such an electrical smoking article is also provided. The cassette resembles a conventional recording tape microcassette, having the web in place of magnetic tape. The cassette may also include the heater past which the tape is indexed to heat individual portions of tobacco flavor medium.

There is a need for an aerosol-generating system that is stable and does not leak liquid. There is a need for a replaceable aerosol-generating article that does not include a heating element.

It is desirable to provide an aerosol-generating system that utilizes a stable aerosol-generating substrate in the replaceable aerosol-generating article. It is desirable to provide the solid aerosol-generating substrate on a tape. The tape may have multiple layers. It is desirable to provide an aerosol-generating system that includes the rotatable heating element in the aerosol-generating device that receives the replaceable aerosol-generating article. It is desirable to provide an aerosol-generating system that includes the rotating heating element having two or more heating surfaces in the aerosol-generating device that receives the replaceable aerosol-generating article. It is desirable to provide an aerosol-generating system that includes a rotating heating element having two or more heating surfaces and includes a heating position and a cleaning position.

This disclosure is directed to an aerosol-generating system that includes the rotating heating element having two or more heating surfaces in the aerosol-generating device that receives the replaceable aerosol-generating article. The replaceable aerosol-generating article includes a solid aerosol-generating substrate on a tape.

According to the present invention, as defined in claim 1, there is provided an aerosol-generating system including an aerosol-generating device and a replaceable cartridge. The aerosol-generating device includes a housing having an air outlet and an inhalation air inlet, an airflow channel fluidly connecting the air outlet with the air inlet, a rotatable heating element coupled to the housing and along the airflow channel, and a cartridge receiving cavity defined withing the housing and configured to receive the cartridge containing a tape. The cartridge is received within the cartridge receiving cavity and the rotatable heating element in contact with the tape. The cartridge includes a rotatable supply reel fixed within the cartridge, a rotatable take-up reel fixed within the cartridge, and the tape disposed on the rotatable supply reel. The tape extends from a first end to a second end. The first end is fixed to the rotatable take-up reel and the second end is fixed to the rotatable supply reel. The tape includes a solid aerosol-generating substrate disposed on a tape. The solid aerosol-generating substrate includes nicotine and glycerol.

According to the present invention, as defined in claim 12, a method of using the aerosol-generating system described herein includes rotating the take-up reel to rotate the rotatable heating element and align a heating surface with the solid aerosol-generating substrate and heating the solid aerosol-generating substrate with the heating surface to form an aerosol in an inhalation airflow.

Advantageously, the aerosol-generating substrate is a solid until the heating step and is thus stable and does not leak liquid during transport or storage of the aerosol-generating substate. In addition, the replaceable replaceable cartridge does not include a heating element and is less complex and less costly to produce. In addition, the replaceable cartridges provide a metered dose of aerosol-generating substrate per inhalation or puff as it is incremented forward by the user. Advantageously, the heating element has two or more heating surfaces or regions about the perimeter and the heating element rotates to provide, at least, a cleaning position and a heating position to maintain the consistent operation of the aerosol-generating system.

All values reported as a percentage are presumed to be weight percent based on the total weight.

All scientific and technical terms used herein have meanings commonly used in the art unless otherwise specified. The definitions provided herein are to facilitate understanding of certain terms used frequently herein.

As used herein, the singular forms "a", "an", and "the" encompass embodiments having plural referents, unless the content clearly dictates otherwise.

As used herein, "or" is generally employed in its sense including "and/or" unless the content clearly dictates otherwise. The term "and/or" means one or all the listed elements or a combination of any two or more of the listed elements.

As used herein, "have", "having", "include", "including", "comprise", "comprising" or the like are used in their open-ended sense, and generally mean "including, but not limited to". It will be understood that "consisting essentially of", "consisting of", and the like are subsumed in "comprising," and the like.

The words "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful and is not intended to exclude other embodiments from the scope of the disclosure, including the claims.

The term "substantially" as used here has the same meaning as "significantly," and can be understood to modify the relevant term by at least about 90 %, at least about 95 %, or at least about 98 %. The term "not substantially" as used here has the same meaning as "not significantly," and can be understood to have the inverse meaning of "substantially," i.e., modifying the relevant term by not more than 10%, not more than 5%, or not more than 2%.

The terms "upstream" and "downstream" refer to relative positions of elements of the inhaler device and inhaler systems described in relation to the direction of inhalation air flow as it is drawn through the body of the inhaler device and inhaler systems.

The term "solid" refers to a fundamental state of matter that does not expand to fill a space or flow, at a temperature of 25 degrees Celsius and one atmosphere pressure.

The term "nicotine" refers to nicotine and nicotine derivatives such as free-base nicotine, nicotine salts and the like.

As used herein, the terms "control electronics", "controller" and "processor" refer to any device or apparatus capable of providing computing capabilities and control capabilities suitable, or configurable to perform the methods, process, and techniques described herein such as, for example, microprocessors, digital signal processors (DSP), application specific integrated circuits (ASIC), field-programmable gate arrays (FPGA), equivalent discrete or integrated logic circuitry, or any combination thereof and of providing suitable data storage capabilities that includes any medium (for example, volatile or non-volatile memory, or magnetic recordable medium such as a disk or tape) containing digital bits (for example, encoded in binary or trinary) that may be readable and writeable.

The term "aerosol" is used here to refer to a suspension of solid particles or liquid droplets, or a combination of solid particles and liquid droplets in a gas. The gas may be air. The solid particles or liquid droplets may comprise one or more volatile flavor compounds. Aerosol may be visible or invisible. Aerosol may include vapors of substances that are ordinarily liquid or solid at room temperature. Aerosol may include vapors of substances that are ordinarily liquid or solid at room temperature, in combination with solid particles or in combination with liquid droplets or in combination with both solid particles and liquid droplets. The aerosol preferably comprises nicotine.

The term "aerosol-generating substrate" is used here to refer to a material capable of releasing one or more volatile compounds that can form an aerosol. In some embodiments, an aerosol-generating substrate may be heated to volatilize one or more components of the aerosol-generating substrate to form an aerosol. In some cases, volatile compounds may be released by a chemical reaction. Aerosol-generating substrate may be solid or liquid or may comprise both solid and liquid components such as a gel. Aerosol-generating substrate may be adsorbed, coated, impregnated, or otherwise loaded onto a carrier or support. Aerosol-generating substrate preferably comprises nicotine. Aerosol-generating substrate may comprise plant-based material. Aerosol-generating substrate may comprise tobacco. Aerosol-generating substrate may comprise a tobacco-containing material containing volatile tobacco flavor compounds, which are released from the aerosol-generating substrate upon heating. Aerosol-generating substrate may alternatively comprise a non-tobacco-containing material. Aerosol-generating substrate may comprise homogenized plant-based material. Aerosol-generating substrate may comprise homogenized tobacco material. Aerosol-generating substrate may comprise at least one aerosol-former. Aerosol-generating substrate may comprise other additives and ingredients, such as flavorants. The aerosol-generating substrate may comprise an active ingredient. The aerosol-generating substrate may be provided as part of an aerosol generating article. The aerosol-generating substrate may be provided in an aerosol-generating article.

The term "aerosol-generating article" is used here to refer to a disposable product capable of including (for example, holding, containing, having, or storing) aerosol-generating substrate. An aerosol-generating article may be capable of removably interfacing, or docking, with an aerosol generating device. This allows the aerosol-generating device to generate aerosol from the aerosol-generating substrate of the aerosol-generating article. An "aerosol-generating cartridge" is an example of an "aerosol-generating article".

The term "aerosol-generating device" is used here to refer to any device configured to be used or utilized with an aerosol forming substrate that releases volatile compounds to form an aerosol that may be inhaled by a user. The aerosol-generating device may be interfaced with an aerosol-generating article comprising the aerosol-generating substrate.

The term "heating element" is used here to refer to any device, apparatus, or portion thereof configured to provide heat, or heat energy, to an aerosol-generating article to release volatile compounds from the aerosol-generating article to form an aerosol.

An aerosol-generating system includes the rotating heating element having two or more heating surfaces in the aerosol-generating device that receives the replaceable aerosol-generating article. The replaceable aerosol-generating article includes a tape having a solid aerosol-generating substrate on the tape. The solid aerosol-generating substrate remains solid until it is heated by the rotating heating element. One of the heating surfaces or regions of the rotatable heating element contacts the solid aerosol-generating substrate and heats the aerosol-generating substrate to a temperature sufficient to vaporize the solid aerosol-generating substrate, preferably in a range from about 200 degrees to about 450 degrees Celsius, or from about 200 degrees to about 350 degrees Celsius. Then the rotatable heating element is rotated to a cleaning position to remove residue from the rotatable heating element surface before it contacts the aerosol-generating substrate again.

An aerosol-generating system including an aerosol-generating device and a replaceable cartridge. The aerosol-generating device includes a housing having an air outlet and an inhalation air inlet, an airflow channel fluidly connecting the air outlet with the air inlet, a rotatable heating element coupled to the housing and along the airflow channel, and a cartridge receiving cavity defined withing the housing and configured to receive the cartridge containing a tape. The cartridge is received within the cartridge receiving cavity and the rotatable heating element in contact with the tape. The cartridge includes a rotatable supply reel fixed within the cartridge, a rotatable take-up reel fixed within the cartridge, and the tape disposed on the rotatable supply reel. The tape extends from a first end to a second end. The first end is fixed to the rotatable take-up reel and the second end is fixed to the rotatable supply reel. The tape includes a solid aerosol-generating substrate disposed on the tape. The solid aerosol-generating substrate includes nicotine and glycerol.

The rotatable heating element includes two or more heating surfaces disposed about a perimeter of the rotatable heating element. The heating surfaces generate heat by resistive heating and contact the solid aerosol-generating substrate to heat the aerosol-generating substrate by conduction. The rotatable heating element or heating surfaces are electrically coupled to a power supply in the aerosol-generating device.

The heating surfaces may be placed on opposing sides of the rotatable heating element. The heating surfaces may be equally spaced apart from each other around the perimeter of the rotatable heating element. Each heating surface defines a discrete heating area that is separated from each heating surface a distance sufficient to prevent heating of one heating surface by an adjacent heating surface by conduction. Each heating surface may have a surface area in a range from about 20 mm² to about 100 mm², or from about 40 mm² to about 80 mm², for example.

In an embodiment, the rotatable heating element comprises three heating surfaces disposed about a perimeter of the rotatable heating element. The three heating surfaces may be equally spaced apart from each other around the perimeter of the rotatable heating element.

The rotatable heating element may have a circular perimeter that is co-axial with an axis of rotation of the heating element. The rotatable heating element may define two or more arcuate sections disposed about a perimeter of the heating element and defining the perimeter of the heating element, and each of the heating surfaces rotate co-axially with the axis of rotation of the heating element.

The heating surface may form a linear or flat surface. The heating surface may form a curved or arcuate surface. The two heating surfaces may each form a linear or flat surface. The two heating surfaces may each form a curved or arcuate surface. The three heating surfaces may each form a linear or flat surface. The three heating surfaces may each form a curved or arcuate surface.

The rotatable heating element may be coupled to the movement of the tape contained within the replaceable cartridge. As the tape advances from the rotatable supply reel to the rotatable take-up reel, the rotatable heating element simultaneously rotates to advance one of the heating surfaces onto the tape to conductively heat the aerosol-generating substrate and form aerosol into inhalation air flowing from the air inlet to the air outlet along the airflow channel.

The rotatable heating element may be coupled to the rotatable supply reel or rotatable take-up reel. The rotatable heating element may be coupled to the rotatable supply reel. The rotatable heating element may be coupled to the rotatable take-up reel.

The rotatable heating element may be mechanically coupled to the rotatable supply reel or rotatable take-up reel. For example, the rotatable heating element may be mechanically coupled to the rotatable supply reel or rotatable take-up reel via one or more gear wheels.

The rotatable heating element may be electronically coupled to the rotatable supply reel or rotatable take-up reel. For example, the rotatable heating element may be electronically coupled to the rotatable supply reel or rotatable take-up reel via the control electronics.

Rotation of the heating element or the supply reel or take-up reel may be accomplished by a driving mechanism. The driving mechanism may be coupled to the supply reel, take-up reel, or the heating element. The driving mechanism may be coupled to the take-up reel to rotate the take-up reel and the supply reel and the heating element. The driving mechanism may be coupled to the heating element to rotate the take-up reel and the supply reel.

The driving mechanism may be a force applied be the user, such as a trigger or lever element. The driving mechanism may be a force applied by a stored energy element. The driving mechanism may be a force applied by a stored energy element being a wound spring.

The driving mechanism may be electrically coupled to a power supply. The driving mechanism may be a force applied by a linear actuator. The driving mechanism may be a force applied by a motor. The driving mechanism may be a force applied by a linear actuator or motor electrically coupled to a power supply.

The driving mechanism may be electrically coupled to control electronics. The control electronics may be electrically coupled to the heating element. The control electronics may be electrically coupled to the heating element and the driving mechanism. Activation of the driving mechanism may activate the heating surface contacting the tape.

The aerosol-generating device may include a controller or control electronics comprising one or more processors (for example, microprocessors). The one or more processors may operate with associated data storage, or memory, for access to processing programs or routines and one or more types of data that may be employed to carry out the illustrative methods. For example, processing programs or routines stored in data storage may include programs or routines for controlling or sensing the heating element, cleaning element, pre-heating element and driving mechanism individually controlling the heating element and driving mechanism, implementing programs or schemes using one or more of the heating elements and driving mechanism, cleaning element, pre-heating element, and the like.

The control electronics may comprise a microprocessor, which may be a programmable microprocessor, a microcontroller, or an application specific integrated chip (ASIC) or other electronic circuitry capable of providing control. The control electronics may comprise further electronic components. The control electronics may be configured to regulate a supply of power to the heating element, driving mechanism, cleaning element, pre-heating element, and the like. Power may be supplied to the heating element continuously following activation of the system or may be supplied intermittently, such as on a puff-by-puff basis. The power may be supplied to the heating element in the form of pulses of electrical power.

The aerosol-generating device includes a power supply for the heating element, driving mechanism, cleaning element, or pre-heating element, or combination thereof. The power source may be a battery, such as a lithium iron phosphate battery, within the device. As an alternative, the power supply may be another form of charge storage device such as a capacitor. The power supply may require recharging and may have a capacity that allows for the storage of enough energy for one or more smoking experiences. For example, the power supply may have sufficient capacity to allow for the continuous generation of aerosol for a period of around six minutes, corresponding to the typical time taken to smoke a conventional cigarette, or for a period that is a multiple of six minutes. In another example, the power supply may have sufficient capacity to allow for a predetermined number of puffs or discrete activations of the heating element.

The computer program products used to implement the processes described herein may be provided using any programmable language, for example, a high-level procedural or object orientated programming language that is suitable for communicating with a computer system. Any such program products may, for example, be stored on any suitable device, for example, a storage media, readable by a general or special purpose program, controller apparatus for configuring and operating the computer when the suitable device is read for performing the procedures described herein. In other words, at least in one embodiment, the aerosol-generating device may be implemented using a non-transitory computer readable storage medium, configured with a computer program, where the storage medium so configured causes the computer to operate in a specific and predefined manner to perform functions described herein.

The exact configuration of the controller of the aerosol-generating device is not limiting and essentially any device capable of providing suitable computing capabilities and control capabilities to implement the method may be used. In view of the above, it will be readily apparent that the functionality may be implemented in any manner as would be known to one skilled in the art. As such, the computer language, the controller, or any other software/hardware which is to be used to implement the processes described herein shall not be limiting on the scope of the systems, processes, or programs (for example, the functionality provided by such processes or programs) described herein. The methods and processes described in this disclosure, including those attributed to the systems, or various constituent components, may be implemented, at least in part, in hardware, software, firmware, or any combination thereof. For example, various embodiments of the techniques may be implemented within one or more processors, including one or more microprocessors, DSPs, ASICs, FPGAs, CPLDs, microcontrollers, or any other equivalent integrated or discrete logic circuitry, as well as any combinations of such components. When implemented in software, the functionality ascribed to the systems, devices, and methods described in this disclosure may be embodied as instructions on a computer-readable medium such as RAM, ROM, NVRAM, EEPROM, FLASH memory, magnetic data storage media, optical data storage media, or the like. The instructions may be executed by one or more processors to support one or more embodiments of the functionality.

The rotatable heating element has two or more positions along the rotational path of the heating element. The rotatable heating element has a heating position and a cleaning position along the rotational path of the heating element.

In the heating position, the heating surface contacts the tape, or aerosol-generating substrate, to conductively heat the aerosol-generating substrate. The heating surface may deflect the tape away from the axis of rotation of the heating element. This may aid in maintaining uniform contact between the heating surface and the tape or aerosol-generating substrate.

In the cleaning position, the heating surface is rotated away from the tape and is spaced apart from the tape. In the cleaning position, the heating surface is cleaned to remove residue from the heating surface. Cleaning of the heating surface may be accomplished by mechanical cleaning or thermal cleaning, for example. The heating surface may be mechanically cleaned by means of a brush element, for example. The heating surface may be thermally cleaned by means of pyrolysis to decompose residue. Thermal cleaning may be accomplished by heating the heating surface to a temperature in a range from 400 degrees to 1000 degrees Celsius. The heating surface may be activated to accomplish thermal cleaning. Alternatively, or in addition, a separate cleaning element may apply heat to the heating surface to accomplish thermal cleaning.

The rotatable heating element may have a third position along the rotational path of the heating element. The third position may be a pre-heating position that is between the cleaning position and the heating position. The heating surface is spaced apart from the tape in the pre-heating position. In the pre-heating position, the heating surface is heated to a pre-heat temperature. The pre-heat temperature may be equal to the heating temperature. The pre-heat temperature may be in a range from 200 degrees to 350 degrees Celsius. The pre-heating position may reduce the time needed to vaporize the aerosol-generating substrate since the heating surface is already at the heating temperature as soon as the heating surface contacts the tape or aerosol-generating substrate.

The rotatable heating element may rotate to the next position as the tape is advanced by a single increment. As the heating surface rotates from the heating position to the cleaning position, the tape may deflect back towards the axis of rotation of the rotating heating element and then deflect away from the axis of rotation of the rotating heating element rotates to introduce the next heating surface back at the heating position.

The rotatable heating element is configured to heat the aerosol-generating substrate to at least 200 degrees Celsius within 300 milliseconds. The rotatable heating element is configured to heat the aerosol-generating substrate to at least 250 degrees Celsius within 200 milliseconds, or within 100 milliseconds, or within 50 milliseconds, or within 25 milliseconds.

The cartridge includes a rotatable supply reel fixed within the cartridge, a rotatable take-up reel fixed within the cartridge, and a tape. The tape extends from a first end to a second end. The first end is fixed to the rotatable take-up reel and the second end is fixed to the rotatable supply reel. The tape includes a solid aerosol-generating substrate disposed on the tape. The solid aerosol-generating substrate includes nicotine and glycerol.

The tape may be formed of a material that is heat resistant. For example, the tape does not decompose, ignite, or melt, at the heating temperature. The tape may be formed of a metal. The tape may be formed of carbon fibre.

The tape has a thickness in a range from about 1 micrometre to about 50 micrometres, or from about 1.5 micrometres to about 25 micrometres, or from about 5 micrometres to about 15 micrometres, or about 10 micrometres. The tape may have a width in a range from 1 to 10 mm, or from 2 to 7 mm, for example.

The aerosol-generating substrate is a solid layer disposed on the tape. The aerosol-generating substrate may have a thickness in a range from 100 micrometres to 750 micrometres, or from 200 micrometres to 500 micrometres.

The aerosol-generating substrate may define discrete areas on the tape. Each discrete area of aerosol-generating substrate may have an area in a range from about 20 mm² to about 100 mm², or from about 40 mm² to about 80 mm², for example.

Each discrete area of aerosol-generating substrate may be separated from each other by a distance sufficient to prevent vaporization of adjacent discrete areas of aerosol-generating substrate during the heating step. Each discrete area of aerosol-generating substrate may be separated from each other by about 1 mm to about 5 mm, or 2 mm to about 5 mm, for example.

A method of using the aerosol-generating system described herein includes rotating the rotatable take-up reel to rotate the rotatable heating element and align a heating surface of the rotating heating element with the solid aerosol-generating substrate and heating the solid aerosol-generating substrate with the heating surface to provide an aerosol in an inhalation airflow along the airflow channel of the aerosol-forming device, delivering the aerosol to a user via the airflow outlet.

The user may then advance the tape and rotatable heating element an increment to align another dose of solid aerosol-forming substrate with the heater, and to provide another dose of aerosol in the inhalation airflow along the airflow channel of the aerosol-generating device. The rotating step advances the tape an increment from the supply reel to the take-up reel and advances one of the heating surfaces of the heating element to the heating position contacting the solid aerosol-generating substrate.

When a cleaning surface is provided on the rotating heating element, the rotating step advances the tape an increment from the supply reel to the take-up reel and advances one of the heating surfaces of the rotatable heating element from the heating position to a cleaning position.

When the rotatable heating element includes a pre-heating position, the rotating step advances the tape an increment from the supply reel to the take-up reel and advances one of the heating surfaces of the rotatable heating element from the cleaning position to a pre-heating position. The rotating step advances the tape an increment from the supply reel to the take-up reel and advances one of the heating surfaces of the rotatable heating element from the pre-heating position to the heating position, contacting the solid aerosol-generating substrate to provide an aerosol in an inhalation airflow along the airflow channel of the aerosol-forming device, delivering the aerosol to a user via the airflow outlet.

The invention is defined in the claims. However, below there is provided a non-exhaustive listing of non-limiting examples. Any one or more of the features of these examples may be combined with any one or more features of another example, embodiment, or aspect described herein.

The Examples will now be further described with reference to the figures in which:
**FIG.** 1 is a schematic cross-sectional diagram of an illustrative aerosol-generating system;
**FIG.** 2 is schematic perspective view of a portion of the tape;
**FIG.** 3 is a schematic side elevation view of an illustrative aerosol-generating system;
**FIG.** 4 is a schematic cross-sectional tip view of the aerosol-generating system of **FIG. 3****;**
**FIG. 5** is a perspective view of the aerosol-generating system of **FIG. 3****;**
**FIG.** 6 is a schematic top elevation view of the rotating heating element;
**FIG.** 7A is a perspective side elevation view of another illustrative aerosol-generating system inserting a cartridge into the aerosol-generating device;
**FIG. 7B** is a perspective side elevation view of the aerosol-generating system of **FIG.** 7A with a cartridge inserted into the aerosol-generating device; and
**FIG.** 7C is a perspective side elevation view of the aerosol-generating system of **FIG.** 7A with a cartridge fully inserted into the aerosol-generating device.

The schematic drawings are not necessarily to scale and are presented for purposes of illustration and not limitation. The drawings depict one or more aspects described in this disclosure. However, it will be understood that other aspects not depicted in the drawing fall within the scope and spirit of this disclosure.

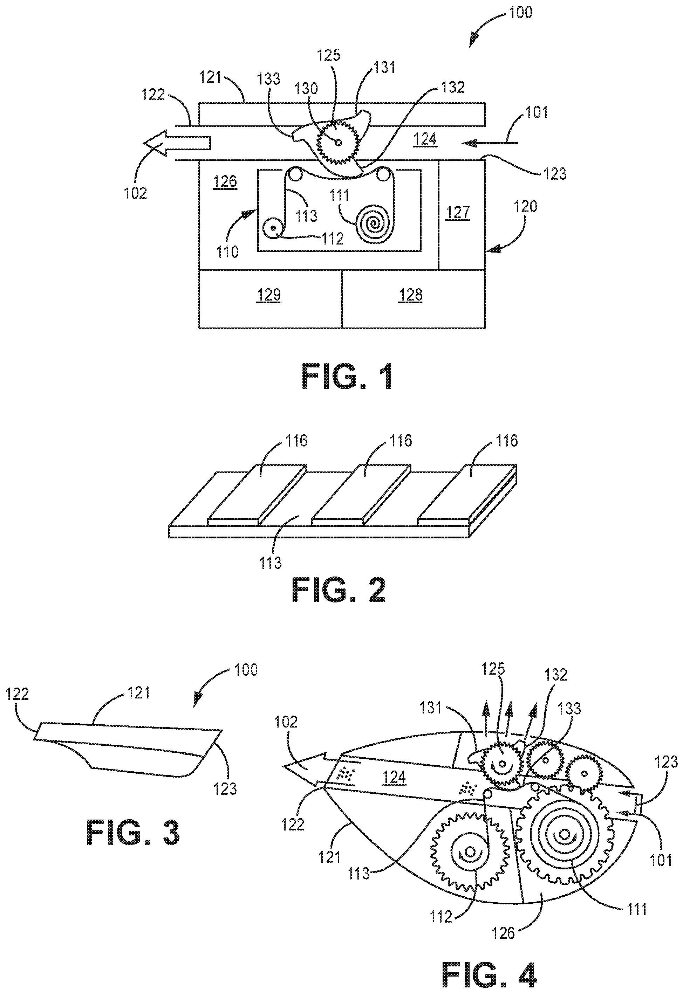

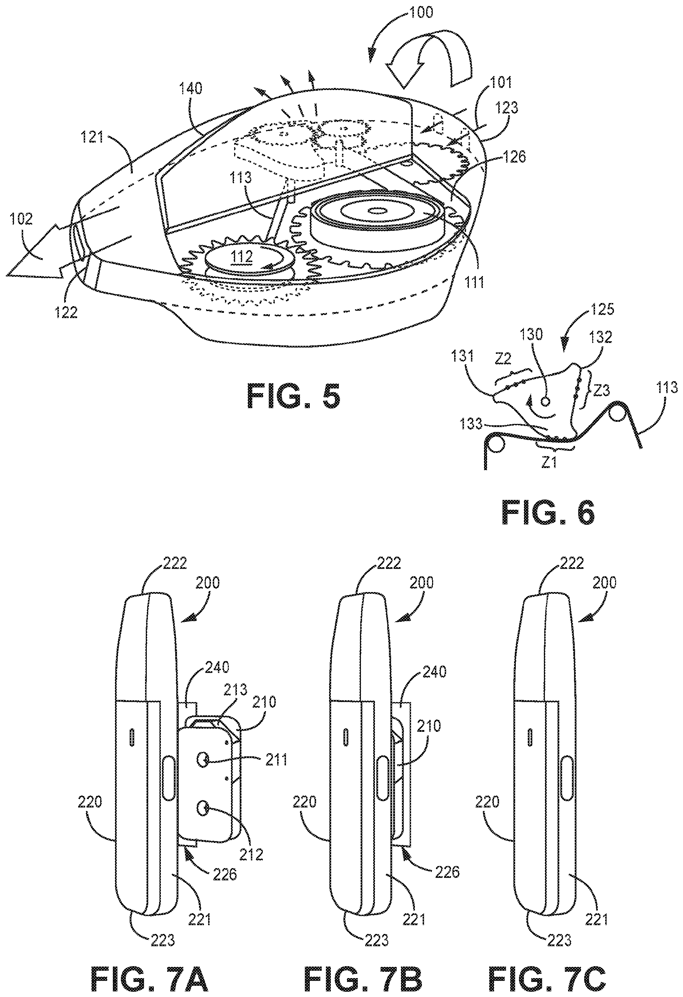

**FIG.** 1 is a schematic cross-sectional diagram of an illustrative aerosol-generating system 100. **FIG.** 2 is schematic perspective view of a portion of the tape 113 with the aerosol-generating substrate 116 spaced apart from each other. **FIG.** 3 is a schematic side elevation view of an illustrative aerosol-generating system **100.** **FIG.** 4 is a schematic cross-sectional tip view of the aerosol-generating system 100 of **FIG. 3****.** **FIG.** 5 is a perspective view of the aerosol-generating system 100 of **FIG.** 3 with an open cartridge lid 140. **FIG.** 6 is a schematic top elevation view of the rotating heating element 125.

The aerosol-generating system 100 includes an aerosol-generating device 120 and a replaceable cartridge 110. The aerosol-generating device 120 includes a housing 121 having an air outlet 122 and an inhalation air inlet 123, an airflow channel 124 fluidly connecting the air outlet 122 with the air inlet 123. Inlet air 101 enters the air inlet 123 and outlet air 102 exists air outlet 122. A rotatable heating element 125 is coupled to the housing 121 and along the airflow channel 124. A cartridge receiving cavity 126 is defined withing the housing 121 and configured to receive the cartridge 110 containing a tape 113. A solid aerosol-generating substrate 116 is disposed on the tape 113. The cartridge 110 is received within the cartridge receiving cavity 126 and the rotatable heating element 125 is in contact with the tape 113. The cartridge 110 includes a rotatable supply reel 111 fixed within the cartridge 110, a rotatable take-up reel 112 fixed within the cartridge 110, and a tape 113 disposed on the rotatable supply reel 111. The tape 113 extends from a first end to a second end. The first end is fixed to the rotatable take-up reel 112 and the second end is fixed to the rotatable supply reel 111. The tape 113 includes a solid aerosol-generating substrate 116 disposed on the tape 113. The solid aerosol-generating substrate 116 includes nicotine and glycerol.

A driving mechanism 127, power supply 128, and control electronics 129 are disposed within the aerosol-generating device 120 housing 121.

The rotatable heating element 125 includes two or more heating surfaces 131, 132, 133 disposed about a perimeter of the rotatable heating element 125. The heating surfaces 131, 132, 133 generate heat by resistive heating and contact the solid aerosol-generating substrate 116 to heat the aerosol-generating substrate 116 by conduction.

The rotatable heating element 125 is coupled to the movement of the tape 113 contained within the replaceable cartridge 110. As the tape 113 advances from the rotatable supply reel 111 to the rotatable take-up reel 112, the rotatable heating element 125 simultaneously rotates to advance one of the heating surfaces 131, 132, 133 onto the tape 113 to conductively heat the aerosol-generating substrate 116 and form aerosol into inhalation air flowing from the air inlet 123 to the air outlet 122 along the airflow channel 124.

The rotatable heating element 125 is coupled to the rotatable supply reel 111 or rotatable take-up reel 112. Rotation of the heating element 125 or the supply reel 111 or take-up reel 112 may be accomplished by a driving mechanism 127. The driving mechanism 127 may be coupled to the supply reel 111, take-up reel 112, or the rotatable heating element 125. The driving mechanism 127 may be coupled to the take-up reel 112 to rotate the take-up reel 112 and the supply reel 111 and the rotatable heating element 125.

The rotatable heating element 125 has two or more positions Z1, Z2, Z3, shown in **FIG. 6** along the rotational path of the heating element 125. The rotatable heating element 125 has a heating position or heating zone Z1 and a cleaning position or cleaning zone Z2 along the rotational path of the heating element 125.

In the heating position or heating zone Z1, the heating surface 133 contacts the tape 113, or aerosol-generating substrate 116, to conductively heat the aerosol-generating substrate 116. The heating surface 133 deflects the tape 113 away from the axis of rotation 130 of the heating element 125.

In the cleaning position or cleaning zone Z2, the heating surface 131 is rotated away from the tape 113 and is spaced apart from the tape 113. In the cleaning position or cleaning zone Z2, the heating surface 131 is cleaned to remove residue from the heating surface 131. Cleaning of the heating surface 131 may be accomplished by mechanical cleaning or thermal cleaning.

The rotatable heating element 125 may have a third position along the rotational path of the heating element 125. The third position may be a pre-heating position or pre-heat zone Z3 that is between the cleaning position or cleaning zone Z2 and the heating position or heating zone Z1. The heating surface 132 is spaced apart from the tape 113 in the pre-heating position or pre-heat zone Z3. In the pre-heating position or pre-heat zone Z3, the heating surface 132 is heated to a pre-heat temperature.

The rotatable heating element 125 rotates to the next position as the tape 113 in advanced by a single increment. As the heating surface 133 rotates from the heating position Z1 to the cleaning position Z2, the tape 113 may deflect back towards the axis of rotation 130 of the heating element 125 and then deflect away from the axis of rotation 130 of the heating element 125 as the next heating surface 132 arrives at the heating position Z1.

**FIG.** 7A is a perspective side elevation view of another illustrative aerosol-generating system 200 inserting a cartridge 210 into the aerosol-generating device 220 with the lid 240 open. **FIG. 7B** is a perspective side elevation view of the aerosol-generating system 200 of **FIG.** 7A with a cartridge 210 inserted into the aerosol-generating device 220 and the lid 240 open. **FIG. 7C** is a perspective side elevation view of the aerosol-generating system 200 of **FIG.** 7A with a cartridge 210 fully inserted into the aerosol-generating device 220 and the lid closed.

The aerosol-generating system 200 including an aerosol-generating device 220 and a replaceable cartridge 210. The aerosol-generating device 220 includes a housing 221 having an air outlet 222 and an inhalation air inlet 223, an airflow channel fluidly connecting the air outlet 222 with the air inlet 223. A rotatable heating element is coupled to the housing 221 and along the airflow channel, and a cartridge receiving cavity 226 is defined withing the housing 221 and configured to receive the cartridge 210 containing a tape 213. A solid aerosol-generating substrate is disposed on the tape. The cartridge 210 is received within the cartridge receiving cavity 226 and the rotatable heating element is in contact with the tape 213. The cartridge 210 includes a rotatable supply reel 211 fixed within the cartridge 210, a rotatable take-up reel 212 fixed within the cartridge 210, and a tape 213. The tape 213 extends from a first end to a second end. The first end is fixed to the rotatable take-up reel 212 and the second end is fixed to the rotatable supply reel 211. The tape 213 includes a solid aerosol-generating substrate disposed on the tape. The solid aerosol-generating substrate includes nicotine and glycerol.

For the purpose of the present description and of the appended claims, except where otherwise indicated, all numbers expressing amounts, quantities, percentages, and so forth, are to be understood as being modified in all instances by the term "about." Also, all ranges include the maximum and minimum points disclosed and include any intermediate ranges therein, which may or may not be specifically enumerated herein. In this context, therefore, a number A is understood as A ±2% of A. Within this context, a number A may be considered to include numerical values that are within general standard error for the measurement of the property that the number A modifies. Also, all ranges include the maximum and minimum points disclosed and include any intermediate ranges therein, which may or may not be specifically enumerated herein.

## Claims

1. An aerosol-generating system (100) comprising:
an aerosol-generating device (120) comprising:
a housing (121) having an air outlet (122) and an air inlet (123);
an airflow channel (124) fluidly connecting the air outlet (122) with the air inlet (123);
a rotatable heating element (125) coupled to the housing (121) and along the airflow channel (124);
a cartridge receiving cavity (126) defined withing the housing (121) and configured to receive a cartridge (110) containing a tape (113);
a cartridge (110) received within the cartridge receiving cavity (126) and the heating element (125) in
contact with the tape (113), the cartridge (110) comprising;
the tape (113) extending from a first end to a second end; a rotatable supply reel (111) fixed within the cartridge (110); a rotatable take-up reel (112) fixed within the cartridge (110); and
the first end of the tape (113) fixed to the rotatable take-up reel (112) and the second end of the tape (113) fixed to the rotatable supply reel (111);
a solid aerosol-generating substrate (116) disposed on the tape (113), the solid aerosol-generating substrate (116) comprising nicotine and glycerol.

2. The aerosol-generating system (100) according to claim 1, wherein the rotatable heating element (125) comprises two or more heating surfaces (131, 132, 133) disposed about a perimeter of the rotatable heating element (125), or the rotatable heating element (125) comprises three heating surface: (131,132,133) disposed about a circumference of the rotatable heating element (125).

3. The aerosol-generating system (100) according to any preceding claim, wherein the rotatable heating element (125) is coupled to the rotatable supply reel (112) or the rotatable take-up reel (111).

4. The aerosol-generating system (100) according to any preceding claim, wherein the rotatable heating element (125) includes a heating position (Z1) and a cleaning position (Z2).

5. The aerosol-generating system (100) according to any preceding claim, wherein the rotatable heating element (125) includes a heating position (Z1), a cleaning position (Z2), and a pre-heating position (Z3).

6. The aerosol-generating system (100) according to claim 2, wherein the rotatable heating element (125) includes a cleaning position (Z2), and in the cleaning position (Z2), the aerosol-generating device (120) includes a mechanically cleaning element configured to contact and clean one of the heating surfaces (131, 132, 133) of the rotatable heating element (125).

7. The aerosol-generating system (100) according to claim 2, wherein the rotatable heating element (125) includes a cleaning position (Z2), and in the cleaning position (Z2), the aerosol-generating device (120) thermally cleans one of the heating surfaces (131, 132, 133) of the rotatable heating element. (125).

8. The aerosol-generating system (100) according to any preceding claim, wherein the rotatable heating element (125) contacts and deflects the tape (113).

9. The aerosol-generating system (100) according to any preceding claim, wherein the aerosol-generating device (120) comprises a power supply (128) electrically coupled to the rotatable heating element (125).

10. The aerosol-generating system (100) according to any preceding claim, wherein the rotatable heating element (125) is configured to heat the aerosol-generating substrate (116) to 200 degrees C within 300 milliseconds.

11. The aerosol-generating system (100) according to any preceding claim, wherein the tape (113) has a thickness in a range from about 1 micrometre to about 50 micrometres, or from about 1.5 micrometres to about 25 micrometres, or from about 5 micrometres to about 15 micrometres, or about 10 micrometres, and aerosol-generating substrate is a solid layer having a thickness in a range from 100 micrometres to 750 micrometres, or from 200 micrometres to 500 micrometres.

12. A method of using the aerosol-generating system (100) according to any preceding claim, comprising:
rotating the rotatable take-up reel (112) to rotate the rotatable heating element (125) and align a heating surface (131, 132, 133) with the solid aerosol-generating substrate (116); and
heating the solid aerosol-generating substrate (116) with the heating surface (131, 132, 133) to form an aerosol in an inhalation airflow.

13. The method of claim 12, wherein the rotating step advances the tape (113) an increment from the rotatable supply reel (111) to the rotatable take-up reel (112) and rotates the rotatable heating element (125) to a heating position wherein the heater of the rotatable heating element (125) contacts the solid aerosol-generating substrate (116).

14. The method of claim 13, wherein the rotating step advances the tape (113) an increment from the rotatable supply reel (111) to the rotatable take-up reel (112) and advances one of the heating surfaces (131,132,133) of the rotatable heating element (125) from the heating position (Z1) to a cleaning position (Z2), and advances one of the heating surfaces (131,132,133) of the rotatable heating element (125) from the cleaning position (Z2) to a pre-heating position (Z3).

15. The method of claim 14, wherein the rotating step advances the tape (113) an increment from the rotatable supply reel (111) to the rotatable take-up reel (112) and advances one of the heating surfaces (131,132,133) of the rotatable heating element (125) from the pre-heating position (Z3) to the heating position (Z1) contacting the solid aerosol-generating substrate (116).

## Patentansprüche

1. Aerosolerzeugungssystem (100), aufweisend:
eine Aerosolerzeugungsvorrichtung (120), aufweisend:
ein Gehäuse (121) mit einem Luftauslass (122) und einem Lufteinlass (123);
einen Luftstromkanal (124), der den Luftauslass (122) mit dem Lufteinlass (123) fluidisch verbindet;
ein drehbares Heizelement (125), das mit dem Gehäuse (121) und entlang des Luftstromkanals (124) gekoppelt ist;
einen Patronenaufnahmehohlraum (126), der innerhalb des Gehäuses (121) definiert und für ein Aufnehmen einer ein Band (113) enthaltenden Patrone (110) ausgelegt ist;
eine Patrone (110), die innerhalb des Patronenaufnahmehohlraums (126) aufgenommen ist, und das Heizelement (125) in Kontakt mit dem Band (113), die Patrone (110) umfassend;
das sich von einem ersten Ende zu einem zweiten Ende erstreckende Band (113);
eine innerhalb der Patrone (110) befestigte drehbare Vorratsspule (111);
eine innerhalb der Patrone (110) befestigte drehbare Aufwickelspule (112); und
das erste Ende des Bandes (113) an der drehbaren Aufnahmespule (112) befestigt ist und das zweite Ende des Bandes (113) an der drehbaren Vorratsspule (111) befestigt ist;
ein festes aerosolerzeugendes Substrat (116), das auf dem Band (113) angeordnet ist, das feste aerosolerzeugende Substrat (116) Nikotin und Glycerin aufweisend.

2. Aerosolerzeugungssystem (100) nach Anspruch 1, wobei das drehbare Heizelement (125) zwei oder mehr Heizflächen (131, 132, 133) aufweist, die um einen Umfang des drehbaren Heizelements (125) angeordnet sind, oder das drehbare Heizelement (125) drei Heizflächen (131, 132, 133) aufweist, die um einen Umfang des drehbaren Heizelements (125) angeordnet sind.

3. Aerosolerzeugungssystem (100) nach einem beliebigen vorhergehenden Anspruch, wobei das drehbare Heizelement (125) mit der drehbaren Vorratsspule (112) oder der drehbaren Aufwickelspule (111) gekoppelt ist.

4. Aerosolerzeugungssystem (100) nach einem beliebigen vorhergehenden Anspruch, wobei das drehbare Heizelement (125) eine Heizposition (Z1) und eine Reinigungsposition (Z2) umfasst.

5. Das Aerosolerzeugungssystem (100) nach einem beliebigen vorhergehenden Anspruch, wobei das drehbare Heizelement (125) eine Heizposition (Z1), eine Reinigungsposition (Z2) und eine Vorheizposition (Z3) umfasst.

6. Aerosolerzeugungssystem (100) nach Anspruch 2, wobei das drehbare Heizelement (125) eine Reinigungsposition (Z2) umfasst und die Aerosolerzeugungsvorrichtung (120) in der Reinigungsposition (Z2) ein mechanisch reinigendes Element umfasst, das ausgelegt ist, um mit einer der Heizflächen (131, 132, 133) des drehbaren Heizelements (125) in Kontakt zu treten und diese zu reinigen.

7. Aerosolerzeugungssystem (100) nach Anspruch 2, wobei das drehbare Heizelement (125) eine Reinigungsposition (Z2) umfasst und die Aerosolerzeugungsvorrichtung (120) in der Reinigungsposition (Z2) eine der Heizflächen (131, 132, 133) des drehbaren Heizelements (125) thermisch reinigt.

8. Aerosolerzeugungssystem (100) nach einem beliebigen vorhergehenden Anspruch, wobei das drehbare Heizelement (125) das Band (113) kontaktiert und ablenkt.

9. Aerosolerzeugungssystem (100) nach einem beliebigen vorhergehenden Anspruch, wobei die Aerosolerzeugungsvorrichtung (120) eine Energieversorgung (128) aufweist, die elektrisch mit dem drehbaren Heizelement (125) gekoppelt ist.

10. Aerosolerzeugungssystem (100) nach einem beliebigen vorhergehenden Anspruch, wobei das drehbare Heizelement (125) ausgelegt ist, um das aerosolerzeugende Substrat (116) innerhalb von 300 Millisekunden auf 200 Grad C zu erwärmen.

11. Aerosolerzeugungssystem (100) nach einem beliebigen vorhergehenden Anspruch, wobei das Band (113) eine Dicke in einem Bereich von etwa 1 Mikrometer bis etwa 50 Mikrometer oder von etwa 1,5 Mikrometer bis etwa 25 Mikrometer oder von etwa 5 Mikrometer bis etwa 15 Mikrometer oder etwa 10 Mikrometer aufweist, und das aerosolerzeugende Substrat eine feste Schicht ist, die eine Dicke in einem Bereich von 100 Mikrometer bis 750 Mikrometer oder von 200 Mikrometer bis 500 Mikrometer aufweist.

12. Verfahren zum Gebrauch des Aerosolerzeugungssystems (100) nach einem beliebigen vorhergehenden Anspruch, umfassend:
Drehen der drehbaren Aufwickelspule (112), um das drehbare Heizelement (125) zu drehen und eine Heizfläche (131, 132, 133) mit dem festen aerosolerzeugenden Substrat (116) auszurichten; und
Erwärmen des festen aerosolerzeugenden Substrats (116) mit der Heizfläche (131, 132, 133) , um ein Aerosol in einem Inhalationsluftstrom zu bilden.

13. Verfahren nach Anspruch 12, wobei der Drehschritt das Band (113) um ein Inkrement von der drehbaren Vorratsspule (111) zu der drehbaren Aufwickelspule (112) vorschiebt und das drehbare Heizelement (125) in eine Heizposition dreht, in der die Heizvorrichtung des drehbaren Heizelements (125) das feste aerosolerzeugende Substrat (116) kontaktiert.

14. Verfahren nach Anspruch 13, wobei der Drehschritt das Band (113) um ein Inkrement von der drehbaren Vorratsspule (111) zu der drehbaren Aufwickelspule (112) vorschiebt und eine der Heizflächen (131, 132, 133) des drehbaren Heizelements (125) von der Heizposition (Z1) zu einer Reinigungsposition (Z2) vorschiebt, und eine der Heizflächen (131, 132, 133) des drehbaren Heizelements (125) von der Reinigungsposition (Z2) zu einer Vorheizposition (Z3) vorschiebt.

15. Verfahren nach Anspruch 14, wobei der Drehschritt das Band (113) um ein Inkrement von der drehbaren Vorratsspule (111) zu der drehbaren Aufwickelspule (112) vorschiebt und eine der Heizflächen (131, 132, 133) des drehbaren Heizelements (125) von der Vorheizposition (Z3) in die Heizposition (Z1) vorschiebt, die das feste aerosolerzeugende Substrat (116) kontaktiert.

## Revendications

1. Système de génération d'aérosol (100) comprenant :
un dispositif de génération d'aérosol (120) comprenant :
un logement (121) ayant une sortie d'air (122) et une entrée d'air (123) ;
un conduit d'écoulement d'air (124) raccordant fluidiquement la sortie d'air (122) à l'entrée d'air (123) ;
un élément de chauffage rotatif (125) couplé au logement (121) et le long du conduit d'écoulement d'air (124) ;
une cavité de réception de cartouche (126) définie au sein du logement (121) et configurée pour recevoir une cartouche (110) contenant un ruban (113) ;
une cartouche (110) reçue au sein de la cavité de réception de cartouche (126) et l'élément de chauffage (125) en contact avec le ruban (113), la cartouche (110) comprenant ;
le ruban (113) s'étendant d'une première extrémité à une deuxième extrémité ;
une bobine débitrice rotative (111) fixée au sein de la cartouche (110) ;
une bobine réceptrice rotative (112) fixée au sein de la cartouche (110) ; et
la première extrémité du ruban (113) apposée sur la bobine réceptrice rotative (112) et la deuxième extrémité du ruban (113) apposée sur la bobine débitrice rotative (111) :
un substrat de génération d'aérosol solide (116) disposé sur le ruban (113), le substrat de génération d'aérosol solide (116) comprenant de la nicotine et du glycérol.

2. Système de génération d'aérosol (100) selon la revendication 1, dans lequel l'élément de chauffage rotatif (125) comprend deux ou plus surfaces chauffantes (131, 132, 133) disposées autour d'un périmètre de l'élément de chauffage rotatif (125), ou l'élément de chauffage rotatif (125) comprend trois surfaces chauffantes (131, 132, 133) disposées autour d'une circonférence de l'élément de chauffage rotatif (125).

3. Système de génération d'aérosol (100) selon l'une quelconque des revendications précédentes, dans lequel l'élément de chauffage rotatif (125) est couplé à la bobine débitrice rotative (112) ou à la bobine réceptrice rotative (111).

4. Système de génération d'aérosol (100) selon l'une quelconque des revendications précédentes, dans lequel l'élément de chauffage rotatif (125) comprend une position de chauffage (Z1) et une position de nettoyage (Z2).

5. Système de génération d'aérosol (100) selon l'une quelconque des revendications précédentes, dans lequel l'élément de chauffage rotatif (125) comprend une position de chauffage (Z1), une position de nettoyage (Z2) et une position de préchauffage (Z3).

6. Système de génération d'aérosol (100) selon la revendication 2, dans lequel l'élément de chauffage rotatif (125) comprend une position de nettoyage (Z2), et dans la position de nettoyage (Z2), le dispositif de génération d'aérosol (120) comprend un élément de nettoyage mécanique configuré pour venir en contact avec et nettoyer l'une des surfaces chauffantes (131, 132, 133) de l'élément de chauffage rotatif (125).

7. Système de génération d'aérosol (100) selon la revendication 2, dans lequel l'élément de chauffage rotatif (125) comprend une position de nettoyage (Z2), et dans la position de nettoyage (Z2), le dispositif de génération d'aérosol (120) nettoie thermiquement l'une des surfaces chauffantes (131, 132, 133) de l'élément de chauffage rotatif (125).

8. Système de génération d'aérosol (100) selon l'une quelconque des revendications précédentes, dans lequel l'élément de chauffage rotatif (125) est en contact avec le ruban (113) et le dévie.

9. Système de génération d'aérosol (100) selon l'une quelconque des revendications précédentes, dans lequel le dispositif de génération **d'aérosol** (120) comprend une alimentation électrique (128) couplée électriquement à l'élément de chauffage rotatif (125) .

10. Système de génération d'aérosol (100) selon l'une quelconque des revendications précédentes, dans lequel l'élément de chauffage rotatif (125) est configuré pour chauffer le substrat de génération d'aérosol (116) jusqu'à 200 degrés Celsius en 300 millisecondes.

11. Système de génération d'aérosol (100) selon l'une quelconque des revendications précédentes, dans lequel le ruban (113) a une épaisseur dans une plage d'environ 1 micromètre à environ 50 micromètres, ou d'environ 1,5 micromètre à environ 25 micromètres, ou d'environ 5 micromètres à environ 15 micromètres, ou **d'environ** 10 micromètres, et un substrat de génération **d'aérosol** est une couche solide ayant une épaisseur dans une plage de 100 micromètres à 750 micromètres, ou de 200 micromètres à 500 micromètres.

12. Procédé d'utilisation du système de génération d'aérosol (100) selon l'une quelconque des revendications précédentes, comprenant :
la rotation de la bobine réceptrice rotative (112) pour faire tourner l'élément de chauffage rotatif (125) et aligner une surface chauffante (131, 132, 133) avec le substrat de génération **d'aérosol** solide (116) ; et
le chauffage du substrat de génération d'aérosol solide (116) avec la surface chauffante (131, 132, 133) pour former un aérosol dans un écoulement d'air d'inhalation.

13. Procédé selon la revendication 12, dans lequel **l'étape** de rotation fait avancer le ruban (113) d'un incrément de la bobine débitrice rotative (111) à la bobine réceptrice rotative (112) et fait tourner **l'élément** de chauffage rotatif (125) jusqu'à une position de chauffage dans lequel le dispositif de chauffage de **l'élément** de chauffage rotatif (125) entre en contact avec le substrat de génération d'aérosol solide (116).

14. Procédé selon la revendication 13, dans lequel l'étape de rotation fait avancer le ruban (113) d'un incrément de la bobine débitrice rotative (111) à la bobine réceptrice rotative (112) et fait avancer l'une des surfaces chauffantes (131, 132, 133) de l'élément de chauffage rotatif (125) depuis la position de chauffage (Z1) jusqu'à une position de nettoyage (Z2), et fait avancer l'une des surfaces chauffantes (131, 132, 133) de l'élément de chauffage rotatif (125) depuis la position de nettoyage (Z2) jusqu'à la position de préchauffage (Z3).

15. Procédé selon la revendication 14, dans lequel l'étape de rotation fait avancer le ruban (113) d'un incrément de la bobine débitrice rotative (111) à la bobine réceptrice rotative (112) et fait avancer l'une des surfaces chauffantes (131, 132, 133) de l'élément de chauffage rotatif (125) depuis la position de préchauffage (Z3) jusqu'à la position de chauffage (Z1) entrant en contact avec le substrat de génération d'aérosol solide (116).
